# EUROPEAN PATENT SPECIFICATION

(11) **EP 3 398 509 B1**
(45) Date of publication and mention of the grant of the patent: **24.02.2021**
(21) Application number: 16881467.1
(22) Date of filing: 26.07.2016
(51) Int. Cl.: A61B 5/026, A61M 1/36, A61B 5/00

(54) **EXTRACORPOREAL CIRCULATION DEVICE PROVIDED WITH CARDIAC FUNCTION MEASUREMENT SYSTEM**
EXTRAKORPORALE KREISLAUFVORRICHTUNG MIT EINEM SYSTEM ZUR MESSUNG DER HERZFUNKTION
DISPOSITIF DE CIRCULATION EXTRACORPORELLE COMPRENANT LEDIT SYSTÈME DE MESURE DE LA FONCTION CARDIAQUE

(30) Priority: 28.12.2015 JP 2015256617
(43) Date of publication of application: 07.11.2018
(73) Proprietor: Terumo Kabushiki Kaisha, Tokyo 151-0072 (JP)
(72) Inventor: KUMANO Koko, Ashigarakami-gun Kanagawa 259-0151 (JP); ISHIMORI Motofumi, Ashigarakami-gun Kanagawa 259-0151 (JP)
(74) Representative: TBK
(86) International application number: PCT/JP2016/071854
(87) International publication number: WO 2017/115487

(56) References cited:
- WO-A1-2012/114545
- JP-A- H05 137 783
- JP-A- S63 143 078
- JP-A- 2013 208 287
- US-A1- 2004 254 473
- DEPNER T A ET AL: "HEMODIALYSIS ACCESS RECIRCULATION MEASURED BY ULTRASOUND DILUTION", ASAIO JOURNAL, LIPPINCOTT WILLIAMS & WILKINS / ASAIO, HAGERSTOWN, MD, US, vol. 41, no. 3, 1 July 1995 (1995-07-01), pages 749-753, XP000542961, ISSN: 1058-2916

## Description

### Technical Field

The present invention relates to an extracorporeal circulator provided with a cardiac function measuring system.

### Background Art

For example, in a case where cardiac surgery of a patient is performed, an extracorporeal circulator is used. The extracorporeal circulator performs extracorporeal blood circulation, auxiliary circulation, or the like in which a pump operates to remove blood from the vein (vena cava) of a patient via a tube, gas in the blood is exchanged through an artificial lung, and then the blood returns to the artery (aorta) of the patient again via the tube.

In general, for example, when a practitioner mounts an extracorporeal circulator on a patient and performs extracorporeal blood circulation or auxiliary circulation, there is a need to connect various other medical instruments to the patient and acquire vital values, such as a blood pressure value and a body temperature, which are pieces of bio-information on the patient.

### Citation List

### Patent Literature

PTL 1: JP-A-4500764

### Summary of Invention

### Technical Problem

However, for example, when a patient is transported by an ambulance at a first-aid site in emergency, or when a patient is treated in a region such as a remote place where medical instruments are insufficiently prepared, even in a case where vital values of the patient are required to be acquired in addition to an extracorporeal circulator, it is difficult to prepare all of those.

In addition, at an actual first-aid site in emergency, even if such medical instruments are available, there is no time to spare to connect a medical device to a patient in many cases.

**In** Depner T.A. et al.: "Hemodialysis Access Recirculation Measured by Ultrasound Dilution", Asaio Journal, Lippincott Williams & Wilkins / Asaio, Hagerstown, MD, US, vol. 41, no. 3, 1 July 1995, pages 749-753, XP000542961, ISSN: 1058-2916**, a pulsation waveform is displayed which indicates a cardiac function of a human body and which is a flow rate fluctuation waveform of blood in extracorporeal circulation, included in a flow rate waveform measured by a flowmeter.**

**An** object of the present invention is to provide a cardiac function measuring system which is capable of easily acquiring a cardiac function (cardiac state) of a patient without being connected to a special device even in a region such as an actual first-aid site in emergency or a remote place, in which medical instruments are insufficiently prepared, and an extracorporeal circulator provided with a cardiac function measuring system.

### Solution to Problem

According to the present invention, there **is provided an extracorporeal circulator for performing extracorporeal circulation of blood of a human body, comprising a cardiac function measuring system, as specified by the appended claims.**

**The cardiac function measuring system includes** a flowmeter configured to measure a flow rate waveform of blood in extracorporeal circulation, a control unit configured to acquire a pulsation waveform which is a flow rate fluctuation waveform of the blood included in the flow rate waveform measured by the flowmeter, and a display unit configured to display the pulsation waveform indicating a cardiac function of the human body in response to a command from the control unit.

According to the configuration, the flowmeter measures a flow rate waveform of blood in extracorporeal circulation, the control unit is configured to acquire a pulsation waveform which is a flow rate fluctuation waveform of blood included in the flow rate waveform measured by the flowmeter, and the display unit is configured to display a pulsation waveform indicating a cardiac function of a human body in response to a command from the control unit.

Therefore, even in a region such as an actual first-aid site in emergency or a remote place, in which medical instruments are insufficiently prepared, the cardiac function measuring system can easily acquire a cardiac function (cardiac state) of a patient in a non-invasive manner with respect to the patient without being connected to a special device, by only mounting the flowmeter on a part in which a flow rate waveform of blood from a human body is obtained while the blood is circulating.

Preferably, the display unit is configured to change a time unit for detecting the flow rate fluctuation waveform and displays a long-term measurement result as a waveform.

According to the configuration, if the flow rate fluctuation waveform is detected and displayed in minutes, a waveform for each pulsation can be acquired. Accordingly, it is possible to grasp a blood pressure value.

Preferably, the display unit includes a waveform display area portion which displays the pulsation waveform, and a lighting notification area portion which is lit to issue a notification of a status of the pulsation waveform displayed in the waveform display area portion.

According to the configuration, the lighting notification area portion of the display unit is lit to be able to notify a practitioner of the status of the pulsation waveform acquired from a patient. Therefore, the practitioner can visually grasp the state of the cardiac function of the patient.

Preferably, the flowmeter is an ultrasound flowmeter and the flowmeter is removably attached to a tube through which the blood circulates.

According to the configuration, the flowmeter need only be attached to the tube through which blood circulates. Therefore, even in a region such as an actual first-aid site in emergency or a remote place, in which medical instruments are insufficiently prepared, it is possible to acquire the cardiac function (cardiac state) of a patient.

**The** extracorporeal circulator is provided with a cardiac function measuring system including a flowmeter configured to measure a flow rate waveform of blood in extracorporeal circulation, a control unit configured to acquire a pulsation waveform which is a flow rate fluctuation waveform of the blood included in the flow rate waveform measured by the flowmeter, and a display unit configured to display the pulsation waveform indicating a cardiac function of the human body in response to a command from the control unit.

According to the configuration, the flowmeter is configured to measure a flow rate waveform of blood from a human body while the blood is circulating, the control unit is configured to acquire a pulsation waveform which is a flow rate fluctuation waveform of blood included in the flow rate waveform measured by the flowmeter, and the display unit is configured to display a pulsation waveform indicating a cardiac function of a human body in response to a command from the control unit. Therefore, even in a region such as an actual first-aid site in emergency or a remote place, in which medical instruments are insufficiently prepared, the cardiac function measuring system can easily acquire a cardiac function (cardiac state) of a patient in a non-invasive manner with respect to the patient without being connected to a special device, by only mounting the flowmeter on a part in which a flow rate waveform of blood from a human body is obtained while the blood is circulating.

### Advantageous Effects of Invention

The present invention can provide the cardiac function measuring system which is capable of easily acquiring a cardiac function (cardiac state) of a patient without being connected to a special device even at an actual first-aid site in emergency, a remote place, or the like, and the extracorporeal circulator provided with a cardiac function measuring system.

### Brief Description of Drawings

[FIG. 1] FIG. 1 is a system diagram illustrating an example of an extracorporeal circulator in which an embodiment of a cardiac function measuring system of the present invention is applied, for example, oxygenation is performed while blood inside the body of a patient is caused to circulate.
[FIG. 2] FIG. 2 is a view illustrating an example of a display unit of a controller illustrated in FIG. 1.
[FIG. 3] FIG. 3 is a view illustrating an example of electrical connection of the display unit, a green light emitting portion, a yellow light emitting portion, a red light emitting portion, and an alarm buzzer.
[FIG. 4] FIG. 4 is a view illustrating an example of an expected waveform in a case where a cardiac beat of a patient P illustrated in FIG. 1 is strong.
[FIG. 5] FIG. 5 is a view illustrating an example of an expected waveform in a case where the cardiac beat of the patient P illustrated in FIG. 1 is weak.
[FIG. 6] FIG. 6 is a view illustrating an example of a fluctuation in flow rate (L/min) of blood with respect to a lapse of time.
[FIG. 7] FIG. 7 is a view conceptually illustrating an example of a change in waveform every three areas.
[FIG. 8] FIG. 8 is an enlarged view illustrating a part T4 of a cardiac hypofunction area T3 in FIG. 7.

### Description of Embodiment

Hereinafter, a preferable embodiment of the present invention will be described in detail with reference to the drawings.

Since the embodiment described below is a suitably specified example of the present invention, the embodiment is subjected to various limitations which are technically preferable. However, the scope of the present disclosure is not limited to the aspects thereof unless otherwise stated in the following description particularly limiting the present invention.

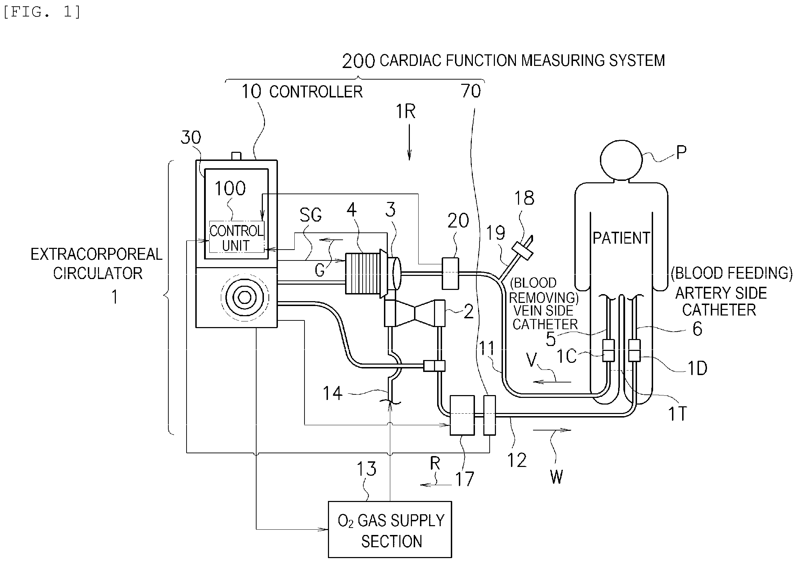

FIG. 1 is a system diagram illustrating an example of an extracorporeal circulator in which an embodiment of a cardiac function measuring system of the present invention is applied so as to perform oxygenation while blood inside the body of a patient is caused to circulate, for example.

A cardiac function measuring system 200 is applied to an extracorporeal circulator 1 illustrated in FIG. 1. "Extracorporeal circulation" performed by an extracorporeal circulator 1 includes an "extracorporeal circulation operation" and an "auxiliary circulation operation". The extracorporeal circulator 1 can perform both the "extracorporeal circulation operation" and the "auxiliary circulation operation".

The "extracorporeal circulation operation" denotes a circulation operation of blood and a gas exchange operation (oxygenation and/or carbon dioxide removal) with respect to the blood performed by the extracorporeal circulator 1 in a case where blood circulation in the heart is temporarily stopped due to cardiac surgery, for example.

In addition, the "auxiliary circulation operation" denotes a circulation operation of blood and a gas exchange operation with respect to the blood which are also performed by the extracorporeal circulator 1 in a case where the heart of a patient P that is an application target of the extracorporeal circulator 1 cannot sufficiently function or in a state where the lung cannot sufficiently perform gas exchange.

In the extracorporeal circulator 1 illustrated in Fig. 1, for example, in a case where cardiac surgery of the patient is performed, a pump of the extracorporeal circulator 1 is operated to remove blood from the vein (vena cava) of the patient, and the blood is oxygenated by exchanging gas in the blood through an artificial lung. Thereafter, it is possible to perform artificial lung extracorporeal blood circulation through which the blood returns to the artery (aorta) of the patient again. The extracorporeal circulator 1 is an apparatus which operates on behalf of a heart and lungs.

As illustrated in FIG. 1, the extracorporeal circulator 1 has a circulation circuit 1R which causes blood to circulate. The circulation circuit 1R includes an artificial lung 2, a centrifugal pump 3, a drive motor 4 which is driving means for driving the centrifugal pump 3, a vein side catheter (blood removing catheter) 5, an artery side catheter (blood feeding catheter) 6, and a controller 10. The controller 10 has a control unit 100.

The centrifugal pump 3 is a so-called rotary pump. While a rotation signal G of the centrifugal pump 3 is transmitted to the controller 10 of the control unit 100, the control unit 100 can determine whether or not the rotational state of the centrifugal pump 3 is steady.

As illustrated in FIG. 1, the vein side catheter (blood removing catheter) 5 is inserted through the femoral vein, and a distal end of the vein side catheter 5 indwells in the right atrium. The artery side catheter (blood feeding catheter) 6 is inserted through the femoral artery. The vein side catheter 5 is connected to the centrifugal pump 3 by using a blood removing tube (also referred to as a blood removing line) 11. The blood removing tube 11 is a conduit line for sending blood.

When the drive motor 4 operates the centrifugal pump 3 in response to a command SG of the controller 10, the centrifugal pump 3 removes blood through the blood removing tube 11 and causes the blood to pass through the artificial lung 2. Thereafter, the centrifugal pump 3 can cause the blood to return to the patient P via a blood feeding tube 12 (also referred to as the blood feeding line).

The artificial lung 2 is disposed between the centrifugal pump 3 and the blood feeding tube 12. The artificial lung 2 performs a gas exchange operation (oxygenation and/or carbon dioxide removal) with respect to blood. The artificial lung 2 is a membrane-type artificial lung, for example. It is particularly preferable to use a hollow fiber membrane-type artificial lung. Oxygen gas is supplied to the artificial lung 2 from an oxygen gas supply section 13 through a tube 14. The blood feeding tube 12 is a conduit line connecting the artificial lung 2 and the artery side catheter 6 to each other.

As the blood removing tube 11 and the blood feeding tube 12, it is possible to use conduit lines made of synthetic resin, for example, vinyl chloride resin or silicone rubber which is highly transparent and flexible to be elastically deformable. Blood (liquid) flows in a V-direction inside the blood removing tube 11, and blood flows in a W-direction inside the blood feeding tube 12.

In an example of the circulation circuit 1R illustrated in FIG. 1, an ultrasound flowmeter 70 which is a preferable example of a flowmeter is removably disposed outside the blood removing tube 11 in a middle part of the blood removing tube 11, for example. The ultrasound flowmeter 70 measures a flow rate of blood flowing inside the blood removing tube 11 in a non-contact manner. As the ultrasound flowmeter 70, for example, an ultrasound propagation time difference-type flowmeter can be used. However, the form thereof is not particularly limited. When not in use, the ultrasound flowmeter 70 can be detached from the blood removing tube 11.

In this way, it is most preferable that the ultrasound flowmeter 70 is removably mounted on the blood removing tube 11. The reason is that the blood removing tube 11 is a tube closest to the heart of the patient P and the blood removing tube 11 can measure the flow rate of blood immediately after blood removal from the heart.

As illustrated in FIG. 1, in the example of the circulation circuit 1R illustrated in FIG. 1, the cardiac function measuring system 200 of the embodiment of the present invention includes the ultrasound flowmeter 70 and the controller 10.

The ultrasound flowmeter 70 illustrated in FIG. 1 generates a blood flow rate measurement signal R when a flow rate of blood flowing inside the blood feeding tube 12 is measured. The blood flow rate measurement signal R is transmitted to the control unit 100, so that the control unit 100 can acquire a flow rate value of blood flowing inside the blood feeding tube 12 at all times.

In the example of the circulation circuit 1R illustrated in FIG. 1, an ultrasound air bubble detection sensor 20 is disposed outside the blood removing tube 11 in a middle part of the blood removing tube 11. A fast clamp 17 is disposed outside the blood feeding tube 12 in an intermediate position of the blood feeding tube 12. In a case where the ultrasound air bubble detection sensor 20 detects that an air bubble is present in blood being sent to the inside of the blood removing tube 11, the ultrasound air bubble detection sensor 20 transmits a measurement signal of air bubble detection to the controller 10. Accordingly, the fast clamp 17 urgently closes the blood feeding tube 12 in response to a command of the controller 10 in order to stop blood from being sent to the patient P side.

In the ultrasound air bubble detection sensor 20, in a case where an air bubble is incorporated into a circuit due to an erroneous operation of a three-way stopcock 18, damage to the tube, or the like during a blood circulation operation, the incorporated air bubble can be detected. If an air bubble is detected, the controller 10 in FIG. 1 sounds an alarm for notification, reduces the rotational frequency of the centrifugal pump 3, or stops the centrifugal pump 3. Moreover, the controller 10 commands the fast clamp 17 such that the fast clamp 17 immediately closes the blood feeding tube 12 and the air bubble is stopped from being sent to the inside of the body of the patient P. Accordingly, the circulation operation of blood in the circulation circuit 1R of the extracorporeal circulator 1 is temporarily halted to prevent an air bubble from being incorporated into the body of the patient P.

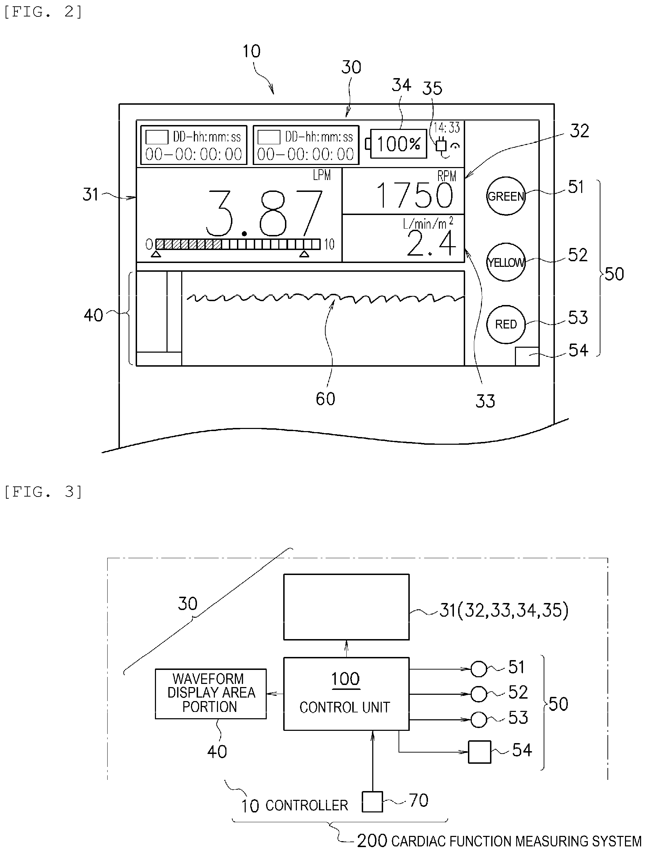

FIG. 2 illustrates an example of a display unit 30 of the controller 10 illustrated in FIG. 1.

The controller 10 illustrated in FIG. 1 has the display unit 30. As illustrated in FIG. 2, The display unit 30 has a blood flow rate display area portion (unit: LPM) 31, a rotational frequency display area portion (unit: RPM) 32 for the centrifugal pump 3, a blood flow rate per minute display area portion (unit: L/min/m2) 33, a waveform display area portion 40, a lighting notification area portion 50, a battery charge state display portion 34 indicating a charged level of a battery, and a power supply connection display portion 35 indicating that the display unit 30 is electrically connected to a commercial power supply.

As the display unit 30, for example, it is possible to use a color liquid crystal display device and an organic EL (electroluminescence) display device.

The blood flow rate display area portion 31 illustrated in FIG. 2 digitally displays a flow rate of blood flowing inside the blood feeding tube 12. The rotational frequency display area portion 32 for the centrifugal pump 3 digitally displays the rotational frequency of the centrifugal pump 3. The blood flow rate per minute display area portion 33 digitally displays an extracorporeal circulation flow rate of blood per 1 m2 in one minute.

The waveform display area portion 40 illustrated in FIG. 2 has a function of displaying the state of a cardiac function of the patient P (which will be described below) in a waveform 60.

The lighting notification area portion 50 illustrated in FIG. 2 has a function of lighting so as to warn a practitioner of the extracorporeal circulator 1 of the status of a state of the cardiac function of the patient P (which will be described below) in three stages, for example. The lighting notification area portion 50 has a green light emitting portion 51, a yellow light emitting portion 52, a red light emitting portion 53, and an alarm buzzer 54. As the green light emitting portion 51, the yellow light emitting portion 52, and the red light emitting portion 53, for example, it is possible to use light emitting diodes emitting colors different from each other.

For example, when the state of the cardiac function is favorable and is in a safety margin, the green light emitting portion 51 emits red light to visually notify the practitioner that the state of the cardiac function is a safety state. When the state of the cardiac function is in a slightly bad condition and is in an alert state while deviating from the safety margin a little, the yellow light emitting portion 52 emits yellow light to visually notify the practitioner that the state of the cardiac function is an alert state. Then, when the state of the cardiac function deviates from the safety margin and is in a critical state, the red light emitting portion 53 emits red light to visually notify the practitioner that the state of the cardiac function is a critical state.

Furthermore, at the same time as the red light emitting portion 53 emits light, the alarm buzzer 54 notifies the practitioner by a sound or voice that the state of the cardiac function is a critical state.

FIG. 3 illustrates an example of electrical connection of the display unit 30, the green light emitting portion 51, the yellow light emitting portion 52, the red light emitting portion 53, and the alarm buzzer 54. As illustrated in FIG. 3, the control unit 100 is electrically connected to the display unit 30, the green light emitting portion 51, the yellow light emitting portion 52, the red light emitting portion 53, and the alarm buzzer 54.

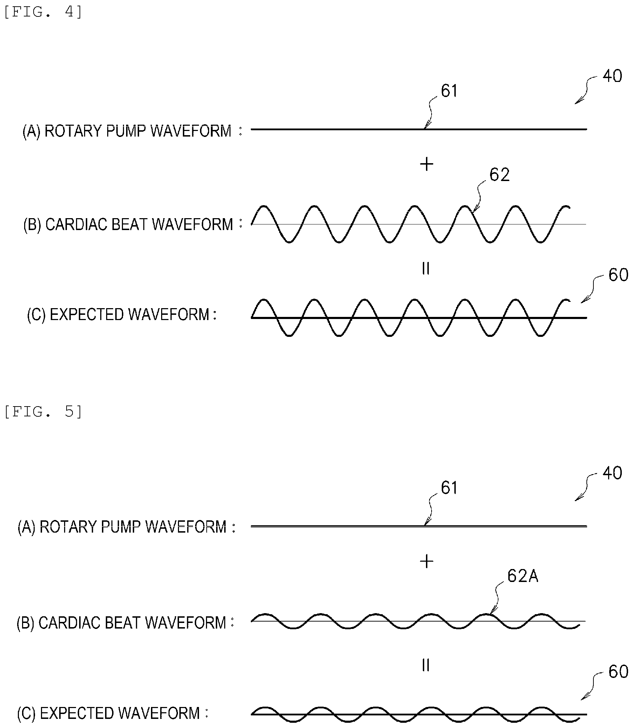

Next, with reference to FIGS. 4 and 5, the waveform 60 displayed in the waveform display area portion 40 illustrated in FIG. 2 will be described. The waveform 60 is a cardiac function display waveform indicating the state of the cardiac function of the patient P.

The waveform 60 illustrated in FIG. 4 illustrates an example of a cardiac function display waveform in a case where a cardiac beat of the patient P illustrated in FIG. 1 is strong. The waveform 60 illustrated in FIG. 5 illustrates an example of a cardiac function display waveform in a case where the cardiac beat of the patient P illustrated in FIG. 1 is weak.

The waveform illustrated in FIG. 4(A) is a rotary pump waveform 61 generated when the centrifugal pump 3 (rotary pump) constantly rotates. The rotary pump waveform 61 is a linear waveform maintaining a constant level.

The waveform illustrated in FIG. 4(B) is a pulsation waveform 62 in a case where the cardiac beat of the patient P is strong. The blood flow rate measurement signal R indicating a flow rate of blood flowing inside the blood feeding tube 12 is transmitted from the ultrasound flowmeter 70 illustrated in FIG. 1 to the control unit 100. The pulsation waveform 62 is a waveform of a pulsation included in the blood flow rate measurement signal R (measurement value) when the flow rate value of blood flowing inside the blood feeding tube 12 is acquired. The waveform 60 illustrated in FIG. 4(C) is a waveform formed by adding the rotary pump waveform 61 illustrated in FIG. 4(A) and the pulsation waveform 62 illustrated in FIG. 4(B).

Similarly, the waveform illustrated in FIG. 5(A) is the rotary pump waveform 61 generated when the centrifugal pump 3 (rotary pump) constantly rotates. The rotary pump waveform 61 is a linear-type waveform maintaining a constant level.

The waveform illustrated in FIG. 5(B) is a pulsation waveform 62A in a case where the cardiac beat of the patient P is weak. The blood flow rate measurement signal R indicating a flow rate of blood flowing inside the blood feeding tube 12 is transmitted from the ultrasound flowmeter 70 illustrated in FIG. 1 to the control unit 100. The pulsation waveform 62A is a waveform of a pulsation included in the blood flow rate measurement signal R (measurement value) when the flow rate value of blood flowing inside the blood feeding tube 12 is acquired. The waveform 60 illustrated in FIG. 5(C) is a waveform formed by adding the rotary pump waveform 61 illustrated in FIG. 5(A) and the pulsation waveform 62 illustrated in FIG. 5(B).

In this way, if the heart of the patient P is weakened when the centrifugal pump 3 constant rotates, the wave height of the pulsation waveform 62A illustrated in FIG. 5 (B) becomes small and gentle compared to the pulsation waveform 62 illustrated in FIG. 4(B).

Meanwhile, in the related art, when the practitioner performs an extracorporeal circulation operation or an auxiliary circulation operation with respect to a patient by using an extracorporeal circulator, there is a need to connect various devices to the patient and acquire vital values, such as a blood pressure value and a body temperature, which are pieces of bio-information on the patient.

However, when a patient is transported by an ambulance at an actual first-aid site in emergency, or when a patient is treated at a place where medical instruments are not ready, such as a remote place, for example, in a case where an extracorporeal circulator is used as described above, it is not possible to connect various medical instruments to the patient and acquire vital values, such as a blood pressure value and a body temperature, which are pieces of bio-information on the patient. In addition, even if such medical instruments are prepared, there is no time to spare to connect medical instruments to a patient at an actual first-aid site in emergency.

Meanwhile, in the embodiment of the present invention, in the display unit 30, the waveform 60 indicating a fluctuation state can be displayed in the waveform display area portion 40 in accordance with the magnitude of the cardiac beat of the patient P. As described below, in regard to displaying this waveform, the time unit in displaying of a measurement result can be increased or reduced in accordance with an instruction of the control unit 100 or switching using an operation button (not separately illustrated) or the like. Accordingly, the practitioner can visually observe the waveform state of the waveform 60 which is displayed in the waveform display area portion 40 at all times. Therefore, the practitioner can visually check magnitude or a fluctuation in the pulsation state of the patient P through the waveform display area portion 40. The practitioner can easily know the cardiac function (cardiac state) of a patient by only observing the waveform display area portion 40 of the display unit 30 of the controller 10 illustrated in FIG. 2.

The pulsation component (fluctuation in flow rate of blood) of the patient P can be reliably acquired by only observing the waveform 60 displayed at all times in the waveform display area portion 40 illustrated in FIG. 2, and the observing substitute for checking a fluctuation in blood pressure of the patient, so that the cardiac function of the patient P can be assumed.

Accordingly, in case of emergency, no line installation time for the patient P is required to measure a blood pressure. Therefore, a practitioner needs only to attach the blood feeding tube 12 from the ultrasound flowmeter 70 in prompt response to the emergency state of the patient P. In addition, there is no need to directly attach a measurement line for measuring the blood pressure and the like of the patient P to the patient P, so that long-term safety of the patient P is improved.

In the extracorporeal circulator 1, for example, even if blood is circulating at an extracorporeal circulation flow rate of 3.87 L/min, the cardiac output amount of the heart is reflected in the ultrasound flowmeter 70. In the related art, it has been considered that the cardiac output amount of the heart is masked (hidden) in the extracorporeal circulation flow rate.

In addition, the practitioner performs an extracorporeal circulation operation or an auxiliary circulation operation of the patient P by using the extracorporeal circulator 1, when the operation ends, the practitioner can visually observe the waveform 60 displayed in the waveform display area portion 40 at all times. Therefore, the practitioner can observe the waveform 60, which is displayed in the waveform display area portion 40 at all times, as a pulsation component (fluctuation in flow rate) of the patient P, and the waveform 60 can substitute for the circumstances of a change in blood pressure of the patient. Therefore, the practitioner can assume the circumstances of the cardiac function of the patient P while observing the waveform 60, so that the practitioner can safely stop the operation of the extracorporeal circulator 1 while watching the circumstances of the cardiac function of the patient P.

Specifically, for example, when the patient P is transported to a medical institution, and after the extracorporeal circulator 1 is attached to the patient P and the patient P is treated, when the practitioner can determine that the waveform 60 displayed in the waveform display area portion 40 illustrated in FIG. 4 as an example is the pulsation waveform 62, for example, in a case where the cardiac beat of the patient P is strong as illustrated in FIG. 4 as an example, the practitioner can determine that the cardiac beat state of the patient P has been able to be ameliorated. Therefore, it is possible for the practitioner to safely separate the extracorporeal circulator 1 from the patient P and end the extracorporeal circulation operation.

In addition, in another specific example, when the patient P is being transported by an ambulance, and after the extracorporeal circulator 1 is attached to the patient P and the patient P is treated while being transported, when the practitioner can assume that the waveform 60 displayed in the waveform display area portion 40 illustrated in FIG. 4 as an example is the pulsation waveform 62, for example, in a case where the cardiac beat of the patient P is strong as illustrated in FIG. 4 as an example, the practitioner can verify that the cardiac state of the patient P has been able to be ameliorated. Therefore, it is possible for the practitioner to safely separate the extracorporeal circulator 1 from the patient P and end the extracorporeal circulation operation.

In this way, in treatment of the patient P using the extracorporeal circulator 1, in the waveform display area portion 40 illustrated in FIG. 4 as an example, when the practitioner can observe the favorable waveform 60 including the pulsation waveform 62 in a case where the cardiac beat of the patient P is strong as illustrated in FIG. 4 as an example, since the cardiac function of the patient P has been strengthened, the practitioner can determine that the treatment has been effective, so that it is possible for the practitioner to safely separate the extracorporeal circulator 1 from the patient P and end the extracorporeal circulation operation.

Next, some preferable examples of lighting display of the lighting notification area portion 50 illustrated in FIG. 2 will be described.

In a case where the favorable waveform 60 illustrated in FIG. 4 as an example is displayed in the waveform display area portion 40 illustrated in FIG. 2, the control unit 100 determines that the cardiac function of the patient P is in the safety margin, thereby causing the green light emitting portion 51 of the lighting notification area portion 50 to be lit. Accordingly, the controller 10 notifies the practitioner that the cardiac function of the patient P is "safe" through the lighting.

In addition, in a case where the waveform 60 illustrated in FIG. 5 as an example is displayed in the waveform display area portion 40 illustrated in FIG. 2, the control unit 100 determines that the cardiac function of the patient P deviates from the safety margin, thereby causing the yellow light emitting portion 52 of the lighting notification area portion 50 to be lit. Accordingly, the controller 10 notifies the practitioner that the cardiac function of the patient P requires "somewhat cautious attention" through the lighting.

Moreover, in a case where a waveform, which has further fallen below the waveform 60 illustrated in FIG. 5 as an example, is displayed in the waveform display area portion 40 illustrated in FIG. 2, for example, in a case where it is assumed that a patient has a symptom such as a myocardial infarction or arrhythmia, the control unit 100 determines that the cardiac function of the patient P completely deviates from the safety margin, thereby causing the red light emitting portion 53 of the lighting notification area portion 50 to be lit and causing the alarm buzzer 54 to generate a warning sound. Accordingly, the controller 10 notifies the practitioner of a "strong warning" for the cardiac function of the patient P through lighting and a sound. In this case, since the controller 10 can notify the practitioner of a "strong warning" through both the lighting and the sound, the practitioner can be more reliably informed.

As described above, the practitioner can grasp the state of the cardiac function of the patient P through a visual sign and a sound from the controller 10.

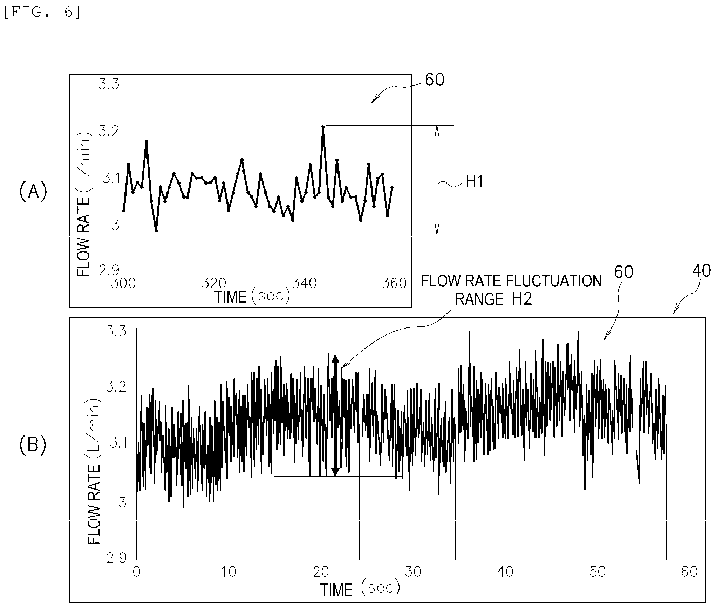

FIGS. 6(A) and 6(B) illustrate an example of a fluctuation in the flow rate (L/min) of blood with respect to a lapse of time in the waveform 60. In FIG. 6(A), the time axis is indicated in seconds, and in FIG. 6(B), the time axis is indicated in minutes.

As illustrated in FIG. 6(A), since the flow rate is measured every second, no change is found in the flow rate for each cardiac beat in a flow rate change range H1. Incidentally, as illustrated in FIG. 6(B), if the time axis is widely taken, that is, in minutes and the entire change in flow rate is seen, it is ascertained that a flow rate fluctuation range H2 is indicated for one pulsation. Consequently, a blood pressure value of the patient can be substantially grasped by adding a liquid feeding pressure and a pressure loss, and the blood pressure value can be approximately calculated based on the numerical values retained in the control unit 100. Since the practitioner is informed of the circumstances at all times, the practitioner can grasp the approximate blood pressure value by observing the waveform 60 displayed in the waveform display area portion 40 at all times.

Therefore, the display unit 30 in FIG. 2 may also display the approximate blood pressure value obtained by such a technique.

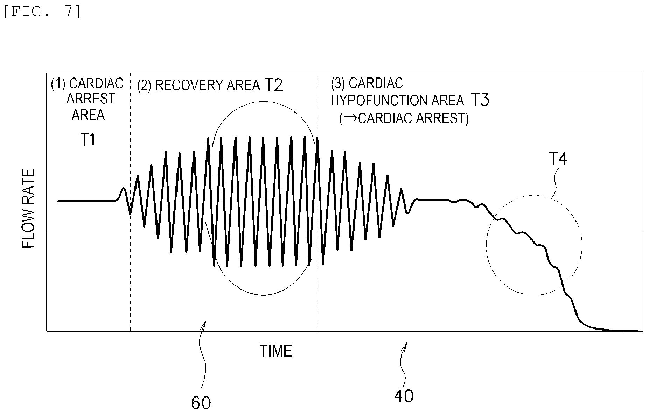

FIG. 7 conceptually illustrates an example of a change in the waveform 60 over three event areas. In FIG. 7, the vertical axis indicates a flow rate of blood, and the horizontal axis indicates a time.

The waveform 60 illustrated in FIG. 7 as an example includes (1) a cardiac arrest area T1, (2) a cardiac function recovery area T2, and (3) a cardiac hypofunction area T3.

First, since no cardiac beat of the patient P is generated in the cardiac arrest area T1, only the rotary pump waveform 61 of the centrifugal pump 3 (blood flow rate of the centrifugal pump 3) is displayed as a linear waveform.

In the ensuing cardiac function recovery area T2, the cardiac beat of the patient P and the cardiac output amount rise, become stable, and are in a pulsation state, so that the cardiac function of the patient P has recovered.

Subsequently, in the cardiac hypofunction area T3 (cardiac arrest), the cardiac output amount is gradually reduced.

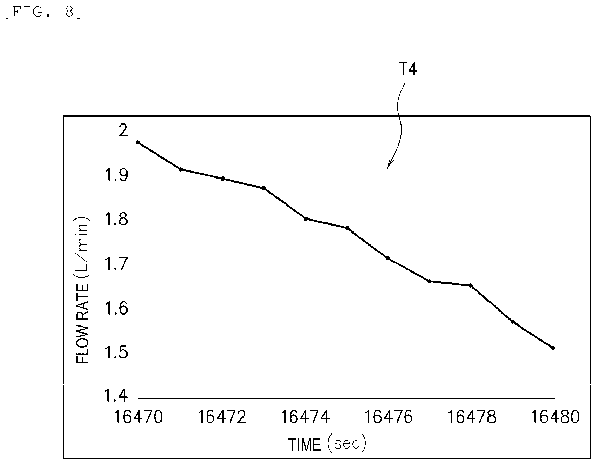

FIG. 8 illustrates an enlarged view of a part T4 of the cardiac hypofunction area T3 in FIG. 7. In FIG. 8, the vertical axis indicates a flow rate (L/min) of blood, and the horizontal axis indicates a time (sec) . In the cardiac hypofunction area T3, almost no cardiac beat is recognized.

As described above, according to the embodiment of the present invention, the cardiac function measuring system 200 includes the flowmeter 70 that measures a flow rate waveform of blood from a human body while the blood is circulating, the control unit 100 that is capable of acquiring the pulsation waveform 62 (waveform 60) which is a flow rate fluctuation waveform of the blood included in the flow rate waveform measured by the flowmeter 70, and the display unit 30 that displays the pulsation waveform 62 (waveform 60) indicating a cardiac function of the human body in response to a command from the control unit 100.

Accordingly, the flowmeter 70 measures the flow rate waveform of blood from the human body while the blood is circulating, and the control unit 100 acquires the pulsation waveform 62 (waveform 60) which is a flow rate fluctuation waveform of the blood included in the flow rate waveform measured by the flowmeter 70. The display unit 30 displays the pulsation waveform 62 (waveform 60) indicating a cardiac function of a human body in response to a command from the control unit 100.

Therefore, even in a region such as an actual first-aid site in emergency or a remote place, in which medical instruments are insufficiently prepared, the cardiac function measuring system 200 can easily acquire a cardiac function (cardiac state) of the patient P in a non-invasive manner with respect to the patient P without being connected to a special device, by only mounting the flowmeter 70 on a part in which a flow rate waveform of blood from a human body is obtained while the blood is circulating.

The display unit 70 includes the waveform display area portion 40 which displays the pulsation waveform 62 (waveform 60), and the lighting notification area portion 50 which is lit to issue a notification of a status of the pulsation waveform displayed in the waveform display area portion 40.

Accordingly, the lighting notification area portion 50 of the display unit 30 is lit to be able to notify a practitioner of the status of the pulsation waveform acquired from a patient. Therefore, the practitioner can easily and visually grasp the state of the cardiac function of the patient P.

The flowmeter 70 is an ultrasound flowmeter and the flowmeter 70 is removably attached to the tube (for example, the blood feeding tube) 12 through which blood circulates. Accordingly, the flowmeter 70 need only be attached to the tube through which blood circulates. Therefore, even in a region such as an actual first-aid site in emergency or a remote place, in which medical instruments are insufficiently prepared, it is possible to acquire the cardiac function (cardiac state) of the patient P.

According to the embodiment of the present invention, the extracorporeal circulator 1 performs extracorporeal circulation of blood of a human body. The extracorporeal circulator is provided with a cardiac function measuring system including the flowmeter 70 that measures a flow rate waveform of blood from a human body while the blood is circulating, the control unit 100 that is capable of acquiring the pulsation waveform 62 (waveform 60) which is a flow rate fluctuation waveform of the blood included in the flow rate waveform measured by the flowmeter 70, and the display unit 30 that displays the pulsation waveform 62 (waveform 60) indicating a cardiac function of the human body in response to a command from the control unit 100.

Accordingly, the flowmeter 70 measures the flow rate waveform of blood from the human body while the blood is circulating, and the control unit 100 acquires the pulsation waveform 62 (waveform 60) which is a flow rate fluctuation waveform of the blood included in the flow rate waveform measured by the flowmeter 70. The display unit 30 displays the pulsation waveform 62 (waveform 60) indicating a cardiac function of a human body in response to a command from the control unit 100.

Therefore, even in a region such as an actual first-aid site in emergency or a remote place, in which medical instruments are insufficiently prepared, the cardiac function measuring system 200 can easily acquire a cardiac function (cardiac state) of the patient P in a non-invasive manner with respect to the patient P without being connected to a special device, by only mounting the flowmeter 70 on a part in which a flow rate waveform of blood from a human body is obtained while the blood is circulating.

The present disclosure is not limited to the above-described embodiment and various changes can be made without departing from the scope of disclosure. The above-described embodiment of the present disclosure can be combined in any manner. Each of the configurations in the embodiment can be partially omitted or can be combined in any manner to be different from that described above.

In the embodiment of the present invention, the cardiac function measuring system 200 is mounted in the extracorporeal circulator 1, and the cardiac function measuring system 200 is configured to have the ultrasound flowmeter 70 and the controller 10. However, the cardiac function measuring system of the present invention is not limited to the extracorporeal circulator 1 and can also be mounted in medical instruments of different types transferring blood through a tube.

### Reference Signs List

- 200: cardiac function measuring system,
- 1: extracorporeal circulator,
- 3: centrifugal pump,
- 10: controller,
- 30: display unit,
- 40: waveform display area portion,
- 50: lighting notification area portion,
- 62: cardiac beat waveform,
- 70: ultrasound flowmeter (flowmeter),
- 100: control unit

## Claims

1. An extracorporeal circulator (1) for performing extracorporeal circulation of blood of a human body, the extracorporeal circulator comprising:
a rotary pump (3) configured to generate a rotary pump waveform (61) by constantly rotating, wherein the rotary pump waveform (61) is a linear-type waveform maintaining a constant level;
a cardiac function measuring system (200) comprising:
a flowmeter (70) configured to measure a flow rate waveform (R) of blood in extracorporeal circulation;
a control unit (100) configured to acquire a waveform (60) that is an addition of a pulsation waveform (62) which is a flow rate fluctuation waveform of the blood included in the flow rate waveform (R) measured by the flowmeter and the rotary pump waveform (61) generated when the rotary pump (3) constantly rotates; and
a display unit (30) configured to display the waveform (60) indicating a cardiac function of the human body in response to a command from the control unit (100);
an artificial lung (2);
a driving means (4) for driving the rotary pump (3);
a blood removing catheter (5); and
a blood feeding catheter (6).

2. The extracorporeal circulator (1) according to Claim 1,
wherein the display unit (30) is configured to change a time unit for detecting the flow rate fluctuation waveform and display a long-term measurement result as a waveform.

3. The extracorporeal circulator (1) according to Claim 1 or 2,
wherein the display unit (30) includes a waveform display area portion which displays the pulsation waveform, and a lighting notification area portion which is lit to issue a notification of a status of the pulsation waveform displayed in the waveform display area portion.

4. The extracorporeal circulator (1) according to any one of Claims 1 to 3,
wherein the flowmeter (70) is an ultrasound flowmeter and the flowmeter is removably attached to a tube through which the blood circulates.

## Patentansprüche

1. Extrakorporalzirkulator (1) zur Durchführung einer extrakorporalen Zirkulation von Blut eines menschlichen Körpers, mit
einer Kreiselpumpe (3), die zur Erzeugung eines Kreiselpumpensignalverlaufs (61) durch konstantes Rotieren eingerichtet ist, wobei der Kreiselpumpensignalverlauf (61) ein Signalverlauf vom linearen Typ ist, der einen konstanten Pegel beibehält,
einem Herzfunktionsmesssystem (200) mit
einem Durchflussmesser (70), der zum Messen eines Flussratensignalverlaufs (R) von Blut bei der extrakorporalen Zirkulation eingerichtet ist,
einer Steuereinheit (100), die zum Beschaffen eines Signalverlaufs (60) eingerichtet ist, der eine Addition eines Pulssignalverlaufs (62), der ein Flussratenschwankungssignalverlauf des Blutes ist, der in dem Flussratensignalverlauf (R) enthalten ist, der durch den Durchflussmesser gemessen wird, und des Kreiselpumpensignalverlaufs (61) ist, der erzeugt wird, wenn die Kreiselpumpe (3) konstant rotiert, und
einer Anzeigeeinheit (30), die zur Anzeige des eine Herzfunktion des menschlichen Körpers angebenden Signalverlaufs (60) im Ansprechen auf einen Befehl von der Steuereinheit (100) eingerichtet ist,
einer künstlichen Lunge (2),
einer Antriebseinrichtung (4) zum Antreiben der Kreiselpumpe (3),
einem Blutabflusskatheter (5) und
einem Blutzuflusskatheter (6).

2. Extrakorporalzirkulator (1) nach Anspruch 1,
wobei die Anzeigeeinheit (30) zur Änderung einer Zeiteinheit zur Erfassung des Flussratenschwankungssignalverlaufs und Anzeige eines Langzeitmessergebnisses als Signalverlauf eingerichtet ist.

3. Extrakorporalzirkulator (1) nach Anspruch 1 oder 2,
wobei die Anzeigeeinheit (30) einen Signalverlaufsanzeigebereichsabschnitt, der den Pulssignalverlauf anzeigt, und einen Leuchtbenachrichtigungsbereichsabschnitt enthält, der zum Ausgeben einer Benachrichtigung eines Status des in dem Signalverlaufsanzeigebereichsabschnitt angezeigten Pulssignalverlaufs eingeschaltet wird.

4. Extrakorporalzirkulator (1) nach einem der Ansprüche 1 bis 3,
wobei der Durchflussmesser (70) ein Ultraschalldurchflussmesser ist und der Durchflussmesser an einer Tuba entfernbar angebracht ist, durch die das Blut zirkuliert.

## Revendications

1. Dispositif de circulation extracorporelle (1) pour réaliser la circulation extracorporelle du sang d'un corps humain, le dispositif de circulation extracorporelle comprenant :
une pompe rotative (3) configurée pour générer une forme d'onde de pompe rotative (61) en tournant de manière constante, dans lequel la forme d'onde de pompe rotative (61) est une forme d'onde de type linéaire maintenant un niveau constant ;
un système de mesure de la fonction cardiaque (200) comprenant :
un débitmètre (70) configuré pour mesurer une forme d'onde de débit (R) du sang dans la circulation extracorporelle ;
une unité de commande (100) configurée pour acquérir une forme d'onde (60) qui est une addition d'une forme d'onde de pulsation (62) qui est une forme d'onde de fluctuation du débit du sang incluse dans la forme d'onde de débit (R) mesurée par le débitmètre et de la forme d'onde de pompe rotative (61) générée lorsque la pompe rotative (3) tourne de manière constante ; et
une unité d'affichage (30) configurée pour afficher la forme d'onde (60) indiquant une fonction cardiaque du corps humain en réponse à une commande provenant de l'unité de commande (100) ;
un poumon artificiel (2) ;
un moyen d'entraînement (4) pour entraîner la pompe rotative (3) ;
un cathéter d'extraction de sang (5) ; et
un cathéter d'alimentation de sang (6).

2. Dispositif de circulation extracorporelle (1) selon la revendication 1,
dans lequel l'unité d'affichage (30) est configurée pour modifier une unité de temps afin de détecter la forme d'onde de fluctuation de débit et afficher un résultat de mesure à long terme en tant que forme d'onde.

3. Dispositif de circulation extracorporelle (1) selon la revendication 1 ou 2,
dans lequel l'unité d'affichage (30) comprend une partie de zone d'affichage de forme d'onde qui affiche la forme d'onde de pulsation, et une partie de zone de notification d'éclairage qui est éclairée pour produire une notification d'un état de la forme d'onde de pulsation affichée dans la partie de zone d'affichage de forme d'onde.

4. Dispositif de circulation extracorporelle (1) selon l'une quelconque des revendications 1 à 3,
dans lequel le débitmètre (70) est un débitmètre à ultrasons et le débitmètre est fixé, de manière amovible, à un tube à travers lequel le sang circule.
